# EUROPEAN PATENT SPECIFICATION

(11) **EP 2 851 096 B1**
(45) Date of publication and mention of the grant of the patent: **28.11.2018**
(21) Application number: 12877065.8
(22) Date of filing: 08.06.2012
(51) Int. Cl.: A61L 27/24, A61L 27/20, A61F 2/30, A61L 27/26, A61K 38/39, A61M 5/31

(54) **COMPOSITION FOR REPAIRING CARTILAGE TISSUE, METHOD FOR PRODUCING SAME, AND USE THEREOF**
ZUSAMMENSETZUNG ZUR REPARATUR VON KNORPELGEWEBE, VERFAHREN ZUR HERSTELLUNG DAVON UND VERWENDUNG DAVON
COMPOSITION POUR RÉPARER UN TISSU CARTILAGINEUX, PROCÉDÉ POUR LA PRODUIRE ET SON UTILISATION

(30) Priority: 16.05.2012 KR 20120052141
(43) Date of publication of application: 25.03.2015
(73) Proprietor: Sewon Cellontech Co., Ltd, Seoul 150-724 (KR)
(72) Inventor: YEO, Se Ken, Yongin-si Gyeonggi-do 446-740 (KR); CHANG, Cheong Ho, Seoul 135-841 (KR); YOO, Ji Chul, Namyangju-si Gyeonggi-do 472-869 (KR); SUH, Dong Sam, Seoul 139-924 (KR); LEE, Jun Keun, Seoul 131-200 (KR); WOO, Sang Hun, Bucheon-si Gyeonggi-do 420-818 (KR)
(74) Representative: dompatent von Kreisler Selting Werner - Partnerschaft von Patent- und Rechtsanwälten mbB
(86) International application number: PCT/KR2012/004524
(87) International publication number: WO 2013/172504

(56) References cited:
- WO-A1-02/36147
- KR-A- 20070 089 490
- KR-A- 20090 131 432
- KR-A- 20100 104 219
- US-A1- 2007 020 225

## Description

### Technical Field

The present invention relates to a composition for cartilage tissue repair, a method for preparing the same, and a method for using the same, and more particularly, a composition based on collagen as a biomaterial is prepared in an injectable form that can be transplanted into cartilage defect regions, and thus, the composition is directly inserted into the application sites through a syringe needle without a surgical incision to enable the promotion of tissue repair, thereby inducing effective cartilage tissue regeneration, and thus inducing relatively easy and prompt cartilage repair and regeneration while reducing surgery-related stress on animals excluding human beings, so that the quality and reliability of a product can be significantly improved, thereby satisfying various desires (needs) of consumers as users thereof and thus giving a good impression to patients.

### Background Art

As well known, cartilage damage is caused by traumatism such as a fall, a direct hit, or a turning force, or disease such as arthritis, osteonecrosis, inflammatory arthritis, or osteochondritis dissecans. Articular cartilage is the tissue that has no neurovascular distribution, is remarkably low in the self-healing capacity compared with the other tissues, and exhibits different effects due to various surgical operations depending on the size and location of lesion.

The articular cartilage is a smooth, extremely slippery, and shiny white material, and is maintained by minimizing friction and wear resistance at the time of joint movement. It has been known that once damaged, the articular cartilage does not regenerate. The damage of cartilage as a tissue in the body is accompanied by severe pain, causing difficulties in daily life, and if chronic, the damaged cartilage proceeds to degenerative arthritis. Therefore, the treatment for regenerating the damaged cartilage tissues needs to be carried out at the time of the damage of cartilage, to prevent the proceeding to degenerative arthritis.

For the treatment of the damaged cartilage defect region, medications injection, physical therapy, intra-articular injection of steroids or hyaluronic acid, or the like is performed as a non-operative treatment method, which only palliates symptoms and pain but has no effect on the treatment of disease. Alternatively, marrow stimulation and side osteochondral transplantation are performed as an operative treatment method. After primary bone stimulation is performed on a site where the cartilage is damaged to be softened or the bone is exposed, the damaged cartilage is repaired, thereby relieving pain and preventing the blood flowing from the bone to synovia from decomposing the synovia and degrading the capacity of synovia. However, the bone stimulation is insufficiently effective, and the hyaluronic acid temporarily reduces pain but does not bring about the induction into appropriate new cartilage tissues. Moreover, osteochondral transplantation requires surgery, and is more difficult and is burdensome in view of cost. Recently, in order to treat the damaged articular cartilage, autologous chondrocyte implantation has been carried out such that the cartilage tissue of a patient is collected to isolate and culture cartilage cells therefrom, which are then again transplanted. This procedure takes a long time to perform two times of operation and tissue collection, and therefore is not easily accessible through the cost burden on patients and health care providers.

Therefore, a novel method of transplanting a biomaterial, which is a mixed composition based on collagen, is required as a simple procedure for the cartilage defect region. This method is useful in treating the cartilage defect region at the early time, and can decrease the number of patients requiring serious surgeries, such as chondrocyte transplantation and artificial joint surgery, afterward.

Recently, there are several attempts in that biomaterials are applied to the cartilage defect region.

The structure of the human body consists of cells and biologically generated polymers. Proteins found in the human body are representative materials. If they are used as natural materials, they can be applied in a form similar to a natural state, and the regeneration of human tissues can be expected. Natural tissues can be used as medical biological tissues, which can provide biological functions that artificial materials cannot, so that the natural tissues are inserted into the human body to provide a biocompatible environment between surrounding tissues, and further function as biological tissues such as natural tissues.

In particular, collagen is a component of structural proteins, and accounts for 1/3 of the overall proteins of a mammal, which constitute soft tissues, such as dermis, tendon/ligament, blood vessel, and cartilage, and hard tissues of the human body.

Collagen is currently used for various medical purposes, such as a styptic, a wound dressing, and wrinkle improvement. Collagen products which have been medically used for a long time have been used without safety problems so far.

Collagen has advantages, such as low antigenicity, high biocompatibility and bioabsorbablity, adhesion to cells, cell growth, cell differentiation induction, blood coagulation, a styptic effect, and biocompatibility with other polymers.

In addition, as a material based on collagen, a composition in which collagen, hyaluronic acid, and a fibrin glue are mixed can exhibit remarkable effects in the repair and regeneration of cartilage.

Hyaluronic acid as a biomaterial is high-viscosity polysaccharide material which is one kind of biopolymer, and has been found in chicken combs, synovial fluid, skin, and the like. The hyaluronic acid has been currently used as an adjurvant for ophthalmology surgery and a treatment agent of arthritis deformans due to high lubricant force.

A product with a hyaluronic acid concentration of 10 mg/mL which is currently available on the market is a joint function improving agent that eliminates the pain caused by gonarthrosis and frozen shoulder disease, and temporarily reduces the pain but causes problems in the case of severe inflammation.

In order to solve the above problems, some prior arts have been filed and registered. That is, prior art 1 has been registered with Korean Patent Registration No. 1114773 (2012.02.02, Patent Application No. 2009-0101388, "Method for Preparing Composition for Articular Cartilage Repairer").

Also, prior art 2 has been published with Korean Patent Publication No. 2004-0008125 (2004.01.28, Patent Application No. 2003-7010108, "Compositions and Methods for Treatment and Repair of Defects or Lesions in Articular Cartilage Using Synovial-Derived Tissue or Cells").

Also, prior art 3 has been registered with Korean Patent Registration No. 684932 (2007.02.13, Patent Application No. 2005-0030837, "Method for Cartilage Regeneration Using Mesenchymal Stem Cells and Ultrasound Stimulation")

WO02/36147A1 discloses a method for preparing a matrix to support the repair of tissue comprising the steps of oxidiying an exogenous polysaccharide to form a modified exogenous polysaccharide having aldehyde groups, and reacting said modified exogenous polysaccharide with mineralized collagen under conditions whereby said aldhyd groups covalently react to crosslink with mineralized collagen to form said matrix.

However, the above-mentioned techniques of the prior art have the following defects.

That is, the prior art has a serious problem that a composition mixed with biocompatible collagen for new cartilage regeneration cannot be provided.

Moreover, the prior art causes inconvenience since a mixed composition is not applied in an injectable form, thereby having a serious problem that damaged cartilage tissue sites cannot be conveniently and effectively stabilized and thus the induction of cartilage regeneration cannot be promoted.

### Detailed Description of the Invention

### Technical Problem

The present invention has been made in order to solve the above-mentioned problems in the prior art, and the present invention provides a composition for cartilage tissue repair, a method for preparing the same, and a method for using the same. Accordingly, a first aspect of the present invention is to provide a composition for cartilage tissue repair, the composition being prepared by mixing collagen having a diluted concentration of 5-60 mg/mL water or a physiological phosphate buffer solution; and hyaluronic acid having a diluted concentration of 5-20 mg/mL water or a physiological phosphate buffer solution using a two-way syringe or a mixer. Through the above-described technical feature, a second aspect of the present invention is that, when a composition based on collagen as a biomaterial is prepared in an injectable form that can be transplanted into cartilage defect regions to promote tissue repair, cartilage tissue regeneration can be effectively induced, thereby inducing relatively easy and prompt cartilage repair and regeneration while reducing surgery-related stress on animals excluding human beings. Further, a third aspect of the present invention is to develop a mixed composition with biocompatible collagen for new cartilage regeneration. In particular, a fourth aspect of the present invention is that the mixed composition is applied in an injectable form, thereby conveniently and effectively stabilizing damaged cartilage tissue sites without surgery. Further, a fifth aspect of the present invention is that the pain due to cartilage defect can be reduced. Further, a sixth aspect of the present invention is that the induction of cartilage regeneration can be promoted. Further, a seventh object of the present invention is that quality and reliability of the product can be significantly improved, thereby satisfying various desires (needs) of consumers as users thereof and thus giving a good impression to patients.

### Technical Solution

In accordance with an aspect of the present invention, there is provided a composition for cartilage tissue repair, the composition being prepared by mixing collagen having a diluted concentration of 5-60 mg/mL water or a physiological phosphate buffer solution; and hyaluronic acid having a diluted concentration of 5-20 mg/mL water or a physiological phosphate buffer solution using a two-way syringe or a mixer.

In accordance with another aspect of the present invention, there is provided a method for preparing a composition for cartilage tissue repair, the method including: preparing collagen having a diluted concentration of 5-60 mg/mL water or a physiological phosphate buffer solution; preparing hyaluronic acid having a diluted concentration of 5-20 mg/mL water or a physiological phosphate buffer solution; mixing the collagen and the hyaluronic acid using a two-way syringe or a mixer; and deaerating the mixed composition for cartilage tissue repair using a centrifuge.

The claimed composition is suitable to be used in a method for for cartilage tissue repair, the method including: fixing a pig leg on a mount, damaging the articular cartilage by using a surgical tool, and then inducing defect regions of 2∼4cm² by using a drill; connecting a syringe needle to a syringe container filled with collagen; and suturing the cut site with a suture thread, and directly injecting the glenoid cavity (a space filled with synovia) of the pig knee with collagen with a concentration of 5 ∼ 60 mg/mL contained in the syringe, hyaluronic acid with a concentration of 5 ∼ 20 mg / mL contained in the syringe, and a composition for cartilage tissue repair in which the collagen and the hyaluronic acid are mixed.

### Advantageous Effects

As set forth above, the present invention provides a composition for cartilage tissue repair, the composition being prepared by mixing collagen having a diluted concentration of 5-60 mg/mL water or a physiological phosphate buffer solution; and hyaluronic acid having a diluted concentration of 5-20 mg/mL water or a physiological phosphate buffer solution using a two-way syringe or a mixer.

Through the above-described technical feature, according to the present invention, when a composition based on collagen as a biomaterial is prepared in an injectable form that can be transplanted into cartilage defect regions, cartilage tissue regeneration can be effectively induced, thereby inducing relatively easy and prompt cartilage repair and regeneration while reducing surgery-related stress on animals excluding human beings.

Further, according to the present invention, a mixed composition with biocompatible collagen for new cartilage regeneration can be provided.

In particular, according to the present invention, the mixed composition is applied in an injectable form, thereby conveniently and effectively stabilizing damaged cartilage tissue sites.

Further, according to the present invention, the pain due to cartilage defect can be reduced.

Further, according to the present invention, the induction of cartilage regeneration can be promoted.

According to the present invention, quality and reliability of the product can be significantly improved due to the above effects, thereby satisfying various desires (needs) of consumers as users thereof and thus giving a good impression to patients. Thus, the present invention is very useful.

### Brief Description of the Drawings

FIG. 1 is an image illustrating the mixing in a composition for cartilage tissue repair.
FIG. 2 is an image illustrating the mixing using a two-way syringe in a composition for cartilage tissue repair.
FIG. 3 is an image illustrating a cartilage repairing material prepared in an injectable type.
FIG. 4 is an image illustrating a cartilage repairing material stained in blue in order to verify post-injection effects.
FIG. 5 is an image illustrating cartilage defects induced for verifying the effect of an injectable cartilage repairing material.
FIG. 6 is an image illustrating the connection of a syringe needle for direct injection into the body.
FIG. 7 is an image illustrating the direct injection into a cartilage defect region of a pig knee.
FIG. 8 is an image illustrating the cartilage defect region filled with a cartilage repairing material as a result of verifying post-injection effects.
FIG. 9 is an image illustrating the filling composition which is conserved without being washed in water.
FIG. 10 is an image illustrating the morphology of a transplantation after the transplantation of a collagen and fibrin glue-mixed composition.

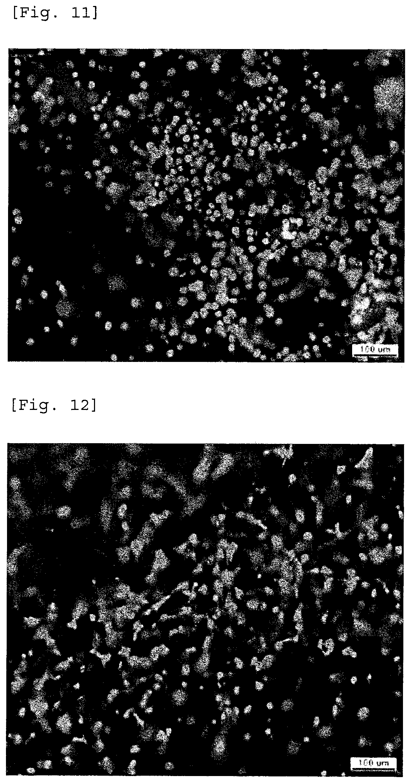

FIGS. 11 and 12 are fluorescent microscopic images illustrating cartilage cell proliferation in a fibrin glue.

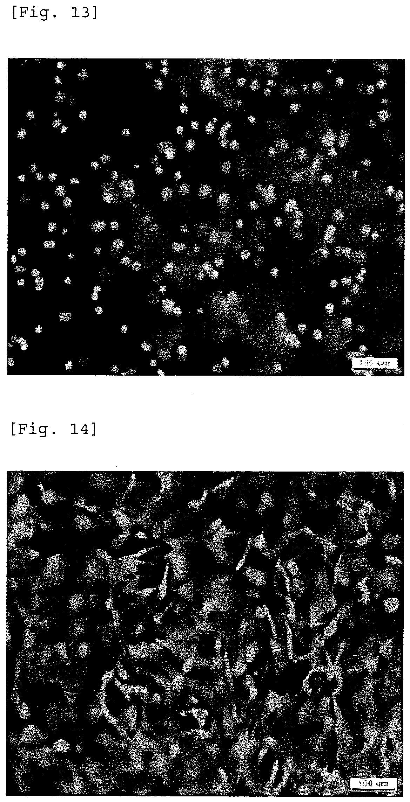

FIGS. 13 and 14 are fluorescent microscopic images illustrating cartilage cell proliferation in a collagen and fibrin glue-mixed composition.

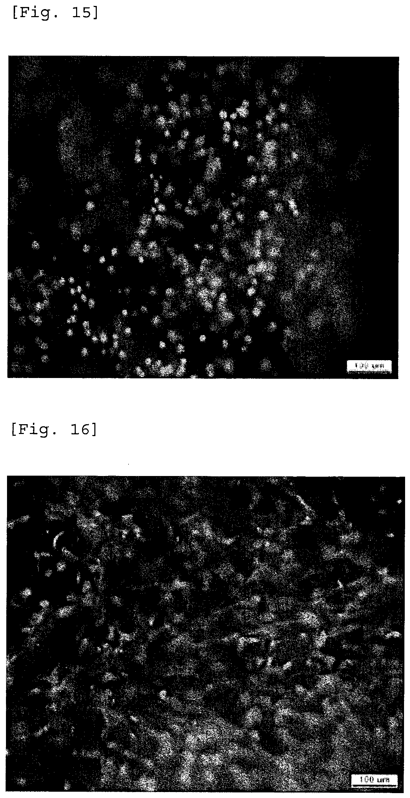

FIGS. 15 and 16 are fluorescent microscopic images illustrating cartilage cell proliferation in a 6% collagen and fibrin glue-mixed composition.

### Mode for Carrying Out the Invention

Hereinafter, a preferred embodiment of the present invention for attaining the above effects will be described in detail with reference to the accompanying drawings.

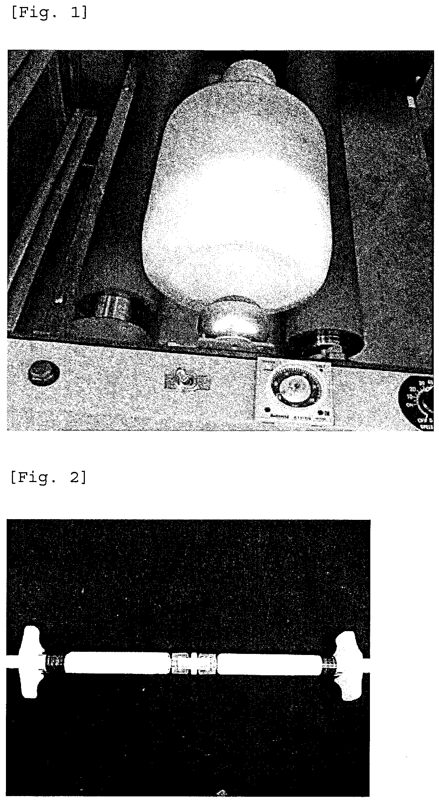

A composition for cartilage tissue repair, a method for preparing the same, and a method for using the same according the present invention are as shown in FIGS. 1 to 10.

In the following descriptions, when it is determined that detailed descriptions of known functions or constitutions associated with the present invention obscure the gist of the present invention, detailed descriptions thereof will be omitted.

In addition, the terms to be later described are defined in consideration of functions in the present invention, and thus the definitions of the terms are to be interpreted throughout the present specification since the terms may be interpreted by the intention of the producer or custom.

First of all, the present invention provides a composition for cartilage tissue repair, the composition being prepared by mixing collagen having a diluted concentration of 5-60 mg/mL water or a physiological phosphate buffer solution; and hyaluronic acid having a diluted concentration of 5-20 mg/mL water or a physiological phosphate buffer solution using a two-way syringe or a mixer.

The composition for cartilage tissue repair is prepared by including the steps: preparing collagen having a diluted concentration of 5-60 mg/mL water or a physiological phosphate buffer solution; preparing hyaluronic acid having a diluted concentration of 5-20 mg/mL water or a physiological phosphate buffer solution; mixing the collagen and the hyaluronic acid using a two-way syringe or a mixer; and deaerating the mixed composition for cartilage tissue repair using a centrifuge.

In particular, centrifugal separation by the centrifuge is characterized by being performed at 2,000-5,000 G while the room temperature is maintained at 1-30 °C.

The composition for cartilage tissue repair prepared as above is suitable to be used (the method of treatment is not part of the invention) by going through the steps: fixing a pig leg on a mount, damaging the articular cartilage by using a surgical tool, and then inducing defect regions of 2∼4cm² by using a drill; connecting a syringe needle to a syringe container filled with collagen; and suturing the cut site with a suture thread, and directly injecting the glenoid cavity (a space filled with synovia) of the pig knee with collagen with a concentration of 5 ∼ 60 mg/mL contained in the syringe, hyaluronic acid with a concentration of 5 ∼ 20 mg / mL contained in the syringe, and a composition for cartilage tissue repair in which the collagen and the hyaluronic acid are mixed.

Meanwhile, according to the present invention, the composition for cartilage tissue repair can be prepared by including the steps of: preparing collagen having a diluted concentration of 5-60 mg/mL water or a physiological phosphate buffer solution; preparing hyaluronic acid having a diluted concentration of 5-20 mg/mL water or a physiological phosphate buffer solution; and preparing a sample in which 60 mg/mL of collagen and 5 mg/mL of hyaluronic acid are mixed at a ratio of 9:1 to 1:9 or a sample in which 60 mg/mL of collagen and 20 mg/mL of hyaluronic acid are mixed at a ratio of 5:5.

The composition suitable for cartilage tissue repair is suitable to be used by going through the steps:
(1) preparing a collagen solution contained in a syringe container and a fibrin glue product;
(2) fixing a pig leg on a mount, damaging the articular cartilage by using a surgical tool, and then inducing defect regions of 2∼4cm² by using a drill;
(3) injecting the collagen product contained in the syringe into the cartilage defect region of the pig knee to fill the cartilage defect region with the collagen product, and allowing the fibrin glue to cover the collagen and then be gelled, thereby firmly fixing the collagen;
(4), on the contrary to step (3), coating the fibrin glue on the cartilage defect region of the pig to be gelled, and then injecting collagen into the fibrin glue gel to fill the cartilage defect region; and
(5) sprinkling sterile injection water downward at a position of 10 cm above the injection site by using a syringe (25 mL), to verify the resistance against the flowing water and observe the gel state.

Further, according to the present invention, the composition for cartilage tissue repair is prepared such that high-viscosity collagen and hyaluronic acid, or a collagen and hyaluronic acid-mixed composition, as a composition for cartilage repair, is injected into a pre-filled syringe, thereby being used in an injectable form.

Further, according to the present invention, the composition for cartilage tissue repair is used such that a syringe needle is connected to an injectable type cartilage injection agent, and the syringe needle is inserted into an application site using the syringe needle without surgical incision, thereby directly injecting the composition for cartilage repair into the glenoid cavity (a space filled with synovia) of the pig knee.

The composition for cartilage tissue repair is suitable to be used such that a specimen is prepared by artificially forming a cartilage defect region of 2∼4cm²; a composition for cartilage repair is stained in blue and injected into the specimen; and then the transplanted site is opened, thereby verifying morphology of the composition filling the cartilage defect region and verifying the water washability of the composition through the degree of adhesion to the cartilage defect region.

However, it shall be noted that it is not intended to limit the present invention to specific embodiments described in the detailed description, but intended to cover all the modifications, equivalents or substitutions belonging to the technical idea and technical scope of the present invention, which are defined by the accompanying claims.

The composition for cartilage tissue repair, the method for preparing the same, and the method for using the same will be described below.

First of all, according to the present invention, when a composition based on collagen as a biomaterial is prepared in a type of an injection capable of being transplanted into cartilage defect regions, cartilage tissue regeneration can be effectively induced, thereby making it possible to reduce surgery-related stress on animals excluding human beings while inducing relatively easy and prompt cartilage repair and regeneration.

Further, not part of the present invention the cartilage defect treatment is possible through a simple procedure, and through a convenient procedure in which simple injection type composition transplantation is performed without surgical incision surgery, the cartilage defect treatment is possible at an early time, thereby decreasing the number of patients requiring joint surgery. Further, the above preventive treatment can lead to a decrease in the number of patients with osteoarthritis.

Examples of the present invention therefor are as follows.

### (Example 1)

### Application of collagen and hyaluronic acid -mixed product to cartilage defect region of animal

Purpose: Verification on applicability of injectable type collagen and hyaluronic acid-mixed composition to cartilage defect region of pig knee
1. Collagen having concentrations of 5, 10, 20, 30, and 60 mg/mL and hyaluronic acid having concentrations of 5, 10, and 20 mg / mL are prepared.

Collagen concentration: For the cartilage repair as a use of the present product, the collagen is required to have a concentration of 5 mg/mL or more in order to keep a state in which the cartilage defect region is filled with collagen. Due to the process characteristics (aseptic preparation) in preparing a product for a medical purpose (injectable form), it is difficult to prepare collagen of above 60 mg/mL with ultra-high viscosity, and actually use and apply such collagen. Due to the reason, the concentration of collagen is set to be 5 mg/L to 60 mg/L inclusive.

Hyaluronic acid concentration: It is difficult to prepare (aseptically prepare) hyaluronic acid of 20 mg/mL for a medical purpose due to natural characteristics of hyaluronic acid (viscosity, etc.), and the mixing of the hyaluronic acid with collagen is not easy. In addition, the hyaluronic acid is required to have a concentration of at least 5 mg/mL in order to maintain the feature (viscosity) required for the product. In addition, the hyaluronic acid added to the collagen base acts on the cartilage site and thus improves the lubrication force, thereby helping the bending of cartilage. Due to the above reasons, the hyaluronic acid is added to collagen, and the concentration of hyaluronic acid is set to be 5 mg/L to 20 mg/L inclusive.

### Preparation of collagen and hyaluronic acid-mixed composition and collagen

### Mixing method A: Method of mixing collagen solution and raw material solution

1) The prepared collagen is diluted with water or a physiological phosphate buffer solution to prepare a collagen solution having a desired concentration.
2) The prepared hyaluronic acid (a powder type) is diluted with water or a physiological phosphate buffer solution to have an appropriate concentration.
3) The prepared solutions are mixed by using a two-way syringe enabling mixing for a short time or using a mixer (rolling) (FIGS. 1 and 2).
4) In the case where the raw materials and solutions used for the mixing are stored in a refrigeration condition (2 ∼ 8°C) in advance, the mixing is easily and smoothly performed.

### Mixing method B: Method of directly mixing raw material (powder) with collagen solution

1) The prepared collagen is diluted with water or a physiological phosphate buffer solution to prepare a collagen solution having a desired concentration.
2) The hyalunoic acid (power type) is put in the collagen solution having an appropriate concentration, followed by mixing.
3) The prepared solution is directly mixed by using a two-way syringe enabling mixing for a short time or using a mixing stick.
4) The above mixing manner is needed to prepare a high-concentration product, and the mixing is easily and smoothly performed while the surrounding environment is maintained at a low temperature at the time of mixing.

### Preparation of composition based on collagen into injectable form

1) Collagen and a mixed composition based on collagen are deaerated using a centrifuge.
2) The centrifuge conditions are that the room temperature (1∼ 30°C) is maintained and the centrifuge effect (G value) is 2,2000 to 5,000 G.
3) A syringe container is filled using a high-viscosity pump. Here, a precaution is taken not to generate empty spaces by gradually raising a nozzle from the bottom upward.
4) A gasket is put in a rear surface of the syringe container using a vacuum to completely seal the syringe, thereby preventing the leakage of a liquid from the syringe container.
5) The filled syringe container is stored at room temperature to conserve the characteristics unique to the collagen (1-30°C).

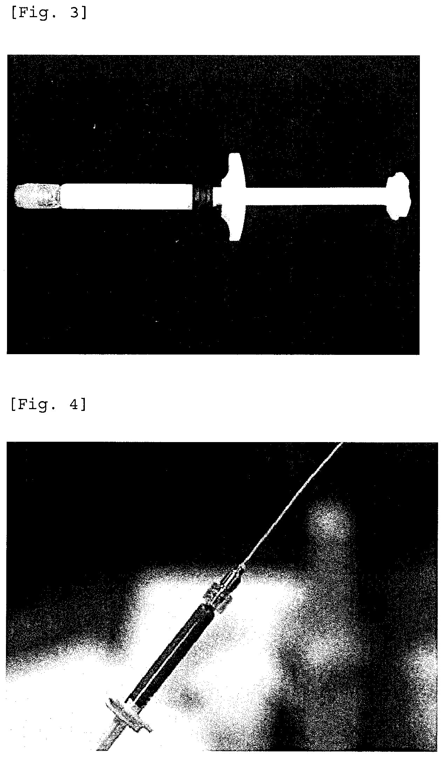

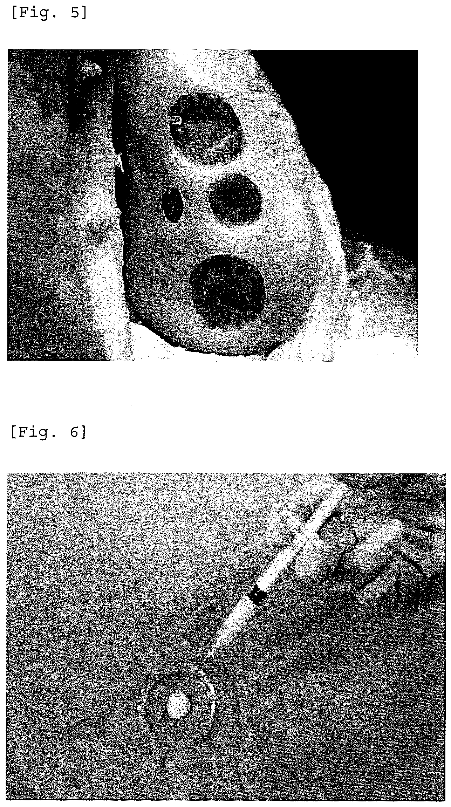

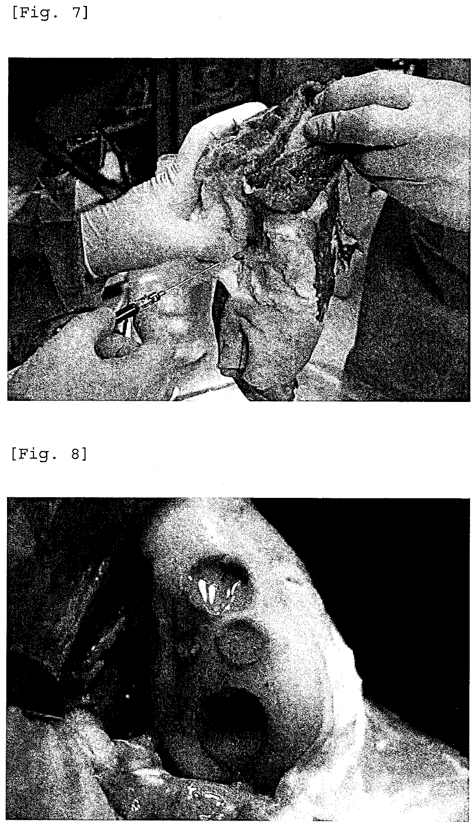

The solution needs to be neither frozen nor denatured by high temperature.
2. For the verification of effect after the injection into the body, a prepared sample is stained with a small quantity of a blue staining agent (Trypan blue, binding to protein) (FIG. 4).
3. Use method and effect verification (not part of the invention)
   1) After a pig leg is fixed on a mount, the articular cartilage of the knee is damaged by using a surgical tool, and large (about 4 cm²) and small (about 2 cm²) defect regions are induced by using a drill (FIG. 5).
   2) A syringe needle is connected to a syringe container filled with collagen or the like. Here, the syringe needle is at least 1 1/2 inch(38 mm) in length.
   3) The cut site is sutured with a suture thread, and the product with each concentration contained in the syringe is directly injected into the glenoid cavity (a space filled with synovia) of the pig knee (FIG. 7).
   4) The knee joint movement (continuous passive motion (CMP)) is performed to promote the action of the injected material which naturally fills the defect regions. In addition, the sutured site is cut to expose the cartilage portion into which the product is injected, for observation.
4. Results: The product injected into the cartilage defect regions naturally fills defect inducing regions, thereby showing an effect preferred by an operator (FIG. 8).

### (Example 2)

### Experiment for verifying adhesiveness of collagen and hyaluronic acid-mixed composition

1. Collagen having concentrations of 10, 30, and 60 mg/mL (1, 3, and 6%) and hyaluronic acid having concentrations of 5, 10, and 20 mg / mL (0.5, 1.0, and 2.0%), which are contained in the syringes, are prepared by the same method as in Example 1. In addition, a sample in which 60 mg/mL of collagen and 5 mg/mL of hyaluronic acid are mixed at a ratio of 9:1 to 1:9 and a sample in which 60 mg/mL of collagen and 20 mg/mL of hyaluronic acid are mixed at a ratio of 5:5 are prepared. Here, the effect of the collagen and hyaluronic acid-mixed composition corresponds to additions of an effect due to characteristics of collagen (biocompatible cartilage components and biodegradation) and an effect due to characteristic of hyaluronic acid (lubrication force), and thus, a composition suitable for cartilage repair is prepared.
2. Measurement of viscosity depending on mixing ratio of collage and hyaluronic acid
The viscosity of each sample is measured by using a Viscometer (DV-1 + PRO).
Measurement item: viscosity
Measurement temperature: 4°C
Measurement RPM: 0.5
Spindle: # SC4-15

**[Table 1]**

| Sample | Viscosity (CP, 10^3) | Note |
|---|---|---|
| 6% collagen | 147 | Maximum viscosity |
| 6% collagen + 0.5% hyaluronic acid (9:1) | 142 | - |
| 6% collagen + 0.5% hyaluronic acid (8:2) | 106 | - |
| 6% collagen + 0.5% hyaluronic acid (7:3) | 79 | - |
| 6% collagen + 0.5% hyaluronic acid (6:4) | 64 | - |
| 6% collagen + 0.5% hyaluronic acid (5:5) | 57 | - |
| 6% collagen + 0.5% hyaluronic acid (4:6) | 46 | - |
| 6% collagen + 0.5% hyaluronic acid (3:7) | 22 | - |
| 6% collagen + 0.5% hyaluronic acid (2:8) | 15 | - |
| 6% collagen + 0.5% hyaluronic acid (1:9) | 3 | - |
| 0.5% hyaluronic acid | 0.5 | Minimum viscosity |

3. Test of adhesiveness depending on mixing ratio of collagen and hyaluronic acid

Adhesiveness comparison is conducted by measuring a material adhesive distance for each sample using a Rheometer (CR-500 DX).

Sample holder moving speed (10 mm/sec)

The moving distance to a site where adhesion is broken during the moving of the sample holder is measured.

**[Table 2]**

| Sample | Material adhesive distance (mm) | Note |
|---|---|---|
| 1% collagen | 19 | - |
| 3% collagen | 47 | - |
| 6% collagen | 61 | Maximum adhesison |
| 6% collagen + 0.5% hyaluronic acid (9:1) | 55 | - |
| 6% collagen + 0.5% hyaluronic acid (8:2) | 49 | - |
| 6% collagen + 0.5% hyaluronic acid (7:3) | 42 | - |
| 6% collagen + 0.5% hyaluronic acid (6:4) | 39 | - |
| 6% collagen + 0.5% hyaluronic acid (5:5) | 36 | - |
| 6% collagen + 0.5% hyaluronic acid (4:6) | 35 | - |
| 6% collagen + 0.5% hyaluronic acid (3:7) | 32 | - |
| 6% collagen + 0.5% hyaluronic acid (2:8) | 25 | - |
| 6% collagen + 0.5% hyaluronic acid (1:9) | 19 | - |
| 6% collagen + 2.0% hyaluronic acid (5:5) | 43 | - |
| 0.5% hyaluronic acid | 13 | Minimum adhesion |
| 1.0% hyaluronic acid | 19 | - |
| 2.0% hyaluronic acid | 23 | - |

Results: The adhesion of the collagen and hyaluronic acid-mixed composition was confirmed, and it was observed that the higher the collagen concentrate, the higher the viscosity.

### (Example 3)

### Test for verifying water washability of collagen and hyaluronic acid after tranplantation thereof

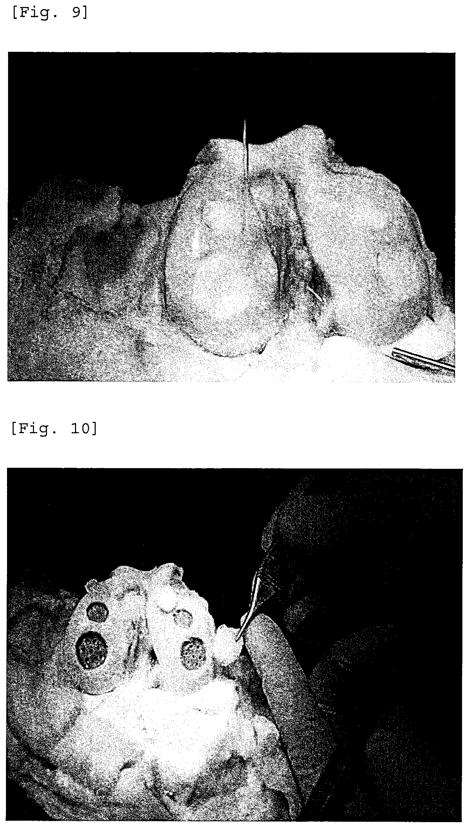

Purpose: Verification on resistance against a factor that interrupts continuous adhering (fixing) and filling of injected product with respect to defect regions
1. Collagen having concentrations of 30 and 60 mg/mL (3 and 6%) and hyaluronic acid having concentrations of 10 and 20 mg/mL (1.0 and 2.0%), which are contained in the syringes, are prepared by the same method as in Example 1.
2. After a pig leg is fixed on a mount, the articular cartilage is damaged by using a surgical tool, and large (about 4 cm²) and small (about 2 cm²) defect regions are induced by using a drill.
3. A product with each concentration filling the syringe is injected into and fills the cartilage defect regions of the pig knee.
4. Injection water is sprinkled downward at a position of 10 cm above the injection site by using a syringe (25 mL).
5. The resistance of the product filling the cartilage defect regions against flowing water is observed by the naked eye.
6. Results: The product filling the cartilage defect regions resisted the flowing water (unwashable), and thus continuously adhered to the defect regions, thereby maintaining a state in which the defect regions were filled with the product (FIG. 9).

### (Example 4) Not part of the present invention

### Application method of collagen and fibrin glue to cartilage defect regions and test on cell proliferation in composition

Purpose: Verification on applicability of injectable type collagen and fibrin glue and on cartilage defect repair by application of collagen and fibrin glue
1. A 6% collagen solution contained in a syringe container is prepared by the same method as in example 1, and a fibrin glue product is prepared. The basic specifications of the fibrin glue produced and used herein are a fibrinogen concentration of 71-127 mg/mL and a thrombin concentration of 400-600 IU/mL (common commercial product specifications).
2. After a pig leg is fixed on a mount, the articular cartilage of the knee is damaged by using a surgical tool, and large (about 4 cm²) and small (about 2 cm²) defect regions are induced by using a drill.
3. The collagen product with a concentration of 6% contained in the syringe is injected into and fills the cartilage defect regions of the pig knee, and then the fibrin glue is allowed to cover the collagen and then gelled, thereby firmly fixing the collagen.
4. On the contrary to item "3", the fibrin glue is coated on the cartilage defect regions of the pig and then gelled, and then 6% collagen is injected into the fibrin glue gel to fill the cartilage defect regions.
5. Sterile injection water is sprinkled downward at a position of 10 cm above the injection site by using a syringe (25 mL), to verify the resistance against the flowing water and thus the gel state.
   Results: The product filling the cartilage defect regions maintained a firm gel form, and resisted the flowing water, and thus continuously adhered to the defect regions, thereby maintaining a state in which the defect regions were filled with the product (FIG. 10).
6. A test on the proliferation of cartilage cells in the collagen and fibrin glue-mixed composition is carried out.

Prepared cartilage cells (1.2 X 10⁷⁰ cells/2mL) are mixed with the mixed composition.

CO₂ culture is carried out at 37°C for 7 days.

The proliferation of cartilage cells through Calcein-AM analysis is observed by a fluorescent microscope.

(From the left, fibrin glue, 3% collagen/fibrin glue mixed composition, and 6% collagen/fibrin glue mixed composition)

**Table 3**

| | | | |
|---|---|---|---|
| Number (Day 0) | FIG. 11 | FIG. 13 | FIG. 15 |
| Day 7 | FIG. 12 | FIG. 14 | FIG. 16 |

Results: As a result of verifying cell proliferation in the composition, it was observed that the cell proliferation was higher in the 3% collagen and fibrin glue-mixed composition and the 6% collagen and fibrin glue-mixed composition rather than in the fibrin glue only.

### Industrial Applicability

Within technical sprits of the composition for cartilage tissue repair, the method for preparing the same, and the use thereof according to the present invention, the same results are substantially reproducible. Particularly, the present invention is carried out to promote the technological development and contribute to the industrial development, and thus the rights of the present invention should be protected under the patent law.

## Claims

1. A composition for cartilage tissue repair, the composition being prepared by mixing collagen having a diluted concentration of 5-60 mg/mL water or a physiological phosphate buffer solution; and hyaluronic acid having a diluted concentration of 5-20 mg/mL water or a physiological phosphate buffer solution using a two-way syringe or a mixer.

2. A method for preparing a composition for cartilage tissue repair, the method comprising: preparing collagen having a diluted concentration of 5-60 mg/mL water or a physiological phosphate buffer solution; preparing hyaluronic acid having a diluted concentration of 5-20 mg/mL water or a physiological phosphate buffer solution; mixing the collagen and the hyaluronic acid using a two-way syringe or a mixer; and deaerating the mixed composition for cartilage tissue repair using a centrifuge.

3. The method of claim 2, wherein centrifugal separation by the centrifuge is performed at 2,000-5,000 G while the room temperature is maintained at 1-30°C.

4. A composition according to claim 1 for use in a method for cartilage tissue repair, the method comprising: fixing a pig leg on a mount, damaging the articular cartilage by using a surgical tool, and then inducing defect regions of 2-4cm² by using a drill; connecting a syringe needle to a syringe container filled with collagen; and suturing the cut site with a suture thread, and directly injecting the glenoid cavity (a space filled with synovia) of the pig knee with collagen with a concentration of 5-60 mg/mL contained in the syringe, hyaluronic acid with a concentration of 5-20 mg/mL contained in the syringe, and a composition for cartilage tissue repair in which the collagen and the hyaluronic acid are mixed.

5. A method for preparing a composition for cartilage tissue repair according to claim 2, the method additionally comprising: preparing a sample in which 60 mg/mL of collagen and 5 mg/mL of hyaluronic acid are mixed at a ratio of 9:1 to 1:9 or a sample in which 60 mg/mL of collagen and 20 mg/mL of hyaluronic acid are mixed at a ratio of 5:5.

6. A composition according to claim 1 for use in a method for cartilage tissue repair, the method comprising: (1) preparing a collagen solution contained in a syringe container and a fibrin glue product; (2) fixing a pig leg on a mount, damaging the articular cartilage by using a surgical tool, and then inducing defect regions of 2-4cm² by using a drill; (3) injecting the collagen product contained in the syringe into the cartilage defect region of the pig knee to fill the cartilage defect region with the collagen product, and allowing the fibrin glue to cover the collagen and then be gelled, thereby firmly fixing the collagen; (4), on the contrary to step (3), coating the fibrin glue on the cartilage defect region of the pig to be gelled, and then injecting collagen into the fibrin glue gel to fill the cartilage defect region; and (5) sprinkling sterile injection water downward at a position of 10 cm above the injection site by using a syringe (25 mL), to verify the resistance against the flowing water and observe the gel state.

7. A method for preparing a composition for cartilage tissue repair according to claim 2, wherein high-viscosity collagen and hyaluronic acid, or a collagen and hyaluronic acid-mixed composition, as a composition for cartilage repair, is injected into a pre-filled syringe, thereby being used in an injectable form.

8. A composition according to claim 1 for use in a method for cartilage tissue repair, wherein a syringe needle is connected to an injectable type cartilage injection agent, and the syringe needle is inserted into an application site using the syringe needle without surgical incision, thereby directly injecting the composition for cartilage repair into the glenoid cavity (a space filled with synovia) of the pig knee.

9. A composition according to claim 1 for use in a method for cartilage tissue repair, wherein a specimen is prepared by artificially forming a cartilage defect region of 2-4cm²; a composition for cartilage repair is stained in blue and injected into the specimen; and then the transplanted site is opened, thereby verifying morphology of the composition filling the cartilage defect region and verifying the water washability of the composition through the degree of adhesion to the cartilage defect region.

## Patentansprüche

1. Zusammensetzung zur Knorpelgewebereparatur, wobei die Zusammensetzung dadurch hergestellt wird, dass man Collagen mit einer verdünnten Konzentration von 5-60 mg/ml Wasser oder einer physiologischen Phosphatpufferlösung und Hyaluronsäure mit einer verdünnten Konzentration von 5-20 mg/ml Wasser oder einer physiologischen Phosphatpufferlösung unter Verwendung einer Zweiwegespritze oder eines Mischers miteinander mischt.

2. Verfahren zur Herstellung einer Zusammensetzung zur Knorpelgewebereparatur, wobei das Verfahren umfasst: Herstellen von Collagen mit einer verdünnten Konzentration von 5-60 mg/ml Wasser oder einer physiologischen Phosphatpufferlösung, Herstellen von Hyaluronsäure mit einer verdünnten Konzentration von 5-20 mg/ml Wasser oder einer physiologischen Phosphatpufferlösung, Mischen des Collagens und der Hyaluronsäure unter Verwendung einer Zweiwegespritze oder eines Mischers; und Entlüften der gemischten Zusammensetzung zur Knorpelgewebereparatur mit Hilfe einer Zentrifuge.

3. Verfahren gemäß Anspruch 2, wobei die zentrifugale Trennung durch die Zentrifuge bei 2000 bis 5000 x g durchgeführt wird, während die Raumtemperatur auf 1-30 °C gehalten wird.

4. Zusammensetzung gemäß Anspruch 1 zur Verwendung in einem Verfahren zur Knorpelgewebereparatur, wobei das Verfahren umfasst: Fixieren eines Schweinebeins auf einem Gestell, Beschädigen des Gelenkknorpels unter Verwendung eines chirurgischen Werkzeugs und dann Induzieren von Defektbereichen von 2-4 cm² unter Verwendung eines Bohrers; Anschließen einer Spritzennadel an einen Spritzenbehälter, der mit Collagen gefüllt ist; und Vernähen der Schnittstelle mit einem chirurgischen Faden und direktes Injizieren von Collagen mit einer Konzentration von 5-60 mg/ml, das in der Spritze enthalten ist, Hyaluronsäure mit einer Konzentration von 5-20 mg/ml, die in der Spritze enthalten ist, und einer Zusammensetzung zur Knorpelgewebereparatur, bei der das Collagen und die Hyaluronsäure miteinander gemischt sind, in die Schulterpfanne (einen mit Synovia gefüllten Raum) des Schweineknies.

5. Verfahren zur Herstellung einer Zusammensetzung zur Knorpelgewebereparatur gemäß Anspruch 2, wobei das Verfahren zusätzlich umfasst: Herstellen einer Probe, bei der 60 mg/ml Collagen und 5 mg/ml Hyaluronsäure in einem Verhältnis von 9:1 bis 1:9 miteinander gemischt sind, oder einer Probe, bei der 60 mg/ml Collagen und 20 mg/ml Hyaluronsäure in einem Verhältnis von 5:5 miteinander gemischt sind.

6. Zusammensetzung gemäß Anspruch 1 zur Verwendung in einem Verfahren zur Knorpelgewebereparatur, wobei das Verfahren umfasst: (1) Herstellen einer Collagenlösung, die in einem Spritzenbehälter enthalten ist, und eines Fibrinkleberprodukts; (2) Fixieren eines Schweinebeins auf einem Gestell, Beschädigen des Gelenkknorpels unter Verwendung eines chirurgischen Werkzeugs und dann Induzieren von Defektbereichen von 2-4 cm² unter Verwendung eines Bohrers; (3) Injizieren des in der Spritze enthaltenen Collagenprodukts in den Knorpeldefektbereich des Schweineknies unter Auffüllen des Knorpeldefektbereichs mit dem Collagenprodukt und Bedecken des Collagens mit dem Fibrinkleber und dann Gelierenlassen des letzteren, wodurch das Collagen fest fixiert wird; (4) im Gegensatz zu Schritt (3) Auftragen des Fibrinklebers auf den Knorpeldefektbereich des Schweineknies, so dass er geliert, und dann Injizieren von Collagen in das Fibrinklebergel unter Auffüllen des Knorpeldefektbereichs; und (5) Hinabspritzen von sterilem Injektionswasser aus einer Position 10 cm oberhalb der Injektionsstelle unter Verwendung einer Spritze (25 ml), um die Beständigkeit gegenüber dem fließenden Wasser zu überprüfen und den Gelzustand zu beobachten.

7. Verfahren zur Herstellung einer Zusammensetzung zur Knorpelgewebereparatur gemäß Anspruch 2, wobei hochviskoses Collagen und Hyaluronsäure oder eine Mischzusammensetzung aus Collagen und Hyaluronsäure als Zusammensetzung zur Knorpelreparatur in eine vorgefüllte Spritze injiziert werden, wodurch sie in einer injizierbaren Form verwendet werden.

8. Zusammensetzung gemäß Anspruch 1 zur Verwendung in einem Verfahren zur Knorpelgewebereparatur, wobei eine Spritzennadel an ein Knorpelinjektionsmittel des injizierbaren Typs angeschlossen wird und die Spritzennadel ohne chirurgischen Einschnitt in eine Applikationsstelle eingeführt wird, wodurch die Zusammensetzung zur Knorpelreparatur direkt in die Schulterpfanne (einen mit Synovia gefüllten Raum) des Schweineknies injiziert wird.

9. Zusammensetzung gemäß Anspruch 1 zur Verwendung in einem Verfahren zur Knorpelgewebereparatur, wobei eine Probe hergestellt wird, indem man künstlich einen Knorpeldefektbereich von 2-4 cm² bildet, eine Zusammensetzung zur Knorpelreparatur wird blau angefärbt und in die Probe injiziert, und dann wird die transplantierte Stelle geöffnet, wobei man die Morphologie der Zusammensetzung, die den Knorpeldefektbereich füllt, überprüft und die Waschbarkeit der Zusammensetzung mit Wasser durch den Grad der Anhaftung an den Knorpeldefektbereich überprüft.

## Revendications

1. Composition pour la réparation de tissu cartilagineux, la composition étant préparée en mélangeant du collagène ayant une concentration diluée de 5-60 mg/ml d'eau ou d'une solution tampon de phosphate physiologique, et de l'acide hyaluronique ayant une concentration diluée de 5-20 mg/ml d'eau ou d'une solution tampon de phosphate physiologique, en utilisant une seringue à deux voies ou un mélangeur.

2. Procédé pour préparer une composition pour la réparation de tissu cartilagineux, le procédé comprenant les étapes consistant à : préparer du collagène ayant une concentration diluée de 5-60 mg/ml d'eau ou d'une solution tampon de phosphate physiologique, préparer de l'acide hyaluronique ayant une concentration diluée de 5-20 mg/ml d'eau ou d'une solution tampon de phosphate physiologique, mélanger le collagène et l'acide hyaluronique en utilisant une seringue à deux voies ou un mélangeur, et désaérer la composition mélangée pour la réparation de tissu cartilagineux en utilisant une centrifugeuse.

3. Procédé selon la revendication 2, dans lequel une séparation centrifuge par la centrifugeuse est effectuée à 2000-5000 x g pendant que la température ambiante est maintenue à 1-30 °C.

4. Composition selon la revendication 1 à utiliser dans un procédé pour la réparation de tissu cartilagineux, le procédé comprenant les étapes consistant à : fixer une jambe de cochon sur un support, endommager le cartilage articulaire en utilisant un outil chirurgical, et ensuite induire des régions de défaut de 2-4 cm² en utilisant une perceuse, relier une aiguille de seringue à un récipient de seringue rempli de collagène, et coudre le site coupé avec un fil de suture, et injecter directement du collagène ayant une concentration de 5-60 mg/ml contenu dans la seringue, de l'acide hyaluronique ayant une concentration de 5-20 mg/ml contenu dans la seringue, et une composition pour la réparation de tissu cartilagineux dans laquelle le collagène et l'acide hyaluronique sont mélangés, dans la cavité glénoïde (un espace rempli de synovie) du genou de cochon.

5. Procédé pour préparer une composition pour la réparation de tissu cartilagineux selon la revendication 2, le procédé comprenant en outre l'étape consistant à : préparer un échantillon dans lequel 60 mg/ml collagène et 5 mg/ml acide hyaluronique sont mélangés à un rapport de 9 : 1 à 1 : 9, ou un échantillon dans lequel 20 mg/ml collagène et 5 mg/ml acide hyaluronique sont mélangés à un rapport de 5 : 5.

6. Composition selon la revendication 1 à utiliser dans un procédé pour la réparation de tissu cartilagineux, le procédé comprenant les étapes consistant à : (1) préparer une solution de collagène contenue dans un récipient de seringue et un produit colle de fibrine, (2) fixer une jambe de cochon sur un support, endommager le cartilage articulaire en utilisant un outil chirurgical, et ensuite induire des régions de défaut de 2-4 cm² en utilisant une perceuse, (3) injecter le produit de collagène contenu dans la seringue dans la région de défaut cartilagineux du genou de cochon pour remplir la région de défaut cartilagineux avec le produit de collagène, et faire la colle de fibrine couvrir le collagène et puis gélifier, ainsi fixant le collagène fermement, (4) au contraire à l'étape (3), revêtir la colle de fibrine sur la région de défaut cartilagineux du cochon pour gélifier, et ensuite injecter du collagène dans le gel de colle de fibrine pour remplir la région de défaut cartilagineux, et (5) saupoudrer de l'eau à injection stérile en bas à partir d'une position de 10 cm au-dessus du site d'injection en utilisant une seringue (25 ml), pour vérifier la résistance contre l'eau coulante et observer l'état du gel.

7. Procédé pour préparer une composition pour la réparation de tissu cartilagineux selon la revendication 2, dans lequel du collagène à haute viscosité et de l'acide hyaluronique, ou une composition mixte de collagène et d'acide hyaluronique, comme composition pour la réparation cartilagineuse est injecté dans une seringue préremplie, ainsi étant utilisée dans une forme injectable.

8. Composition selon la revendication 1 à utiliser dans un procédé pour la réparation de tissu cartilagineux, dans lequel une aiguille de seringue est reliée à un agent d'injection cartilagineuse du type injectable, et l'aiguille de seringue est insérée dans un site d'application en utilisant l'aiguille de seringue sans incision chirurgicale, ainsi injectant la composition pour la réparation cartilagineuse directement dans la cavité glénoïde (un espace rempli de synovie) du genou de cochon.

9. Composition selon la revendication 1 à utiliser dans un procédé pour la réparation de tissu cartilagineux, dans lequel un échantillon est préparé en formant artificiellement une région de défaut cartilagineux de 2-4 cm², une composition pour la réparation de tissu cartilagineux est teintée en bleu et injectée dans l'échantillon, et puis le site transplanté est ouvert, ainsi vérifiant la morphologie de la composition remplissant la région de défaut cartilagineux et vérifiant la lavabilité à l'eau de la composition au moyen du degré de l'adhésion à la région de défaut cartilagineux.
